(19) Europäisches Patentamt
European Patent Office
Office européen des brevets

(11) **EP 0 885 005 B1**

(12) **EUROPÄISCHE PATENTSCHRIFT**

(45) Veröffentlichungstag und Bekanntmachung des Hinweises auf die Patenterteilung:
**27.08.2003 Patentblatt 2003/35**

(51) Int Cl.[7]: **A61K 31/565**, A61P 5/26

(21) Anmeldenummer: **97920511.9**

(22) Anmeldetag: **06.03.1997**

(86) Internationale Anmeldenummer:
**PCT/DE97/00518**

(87) Internationale Veröffentlichungsnummer:
**WO 97/032588 (12.09.1997 Gazette 1997/39)**

(54) **KOMBINATION VON DEHYDROEPIANDROSTERON UND AROMATASEHEMMERN UND VERWENDUNG DIESER KOMBINATION ZUR HERSTELLUNG EINES ARZNEIMITTELS ZUR BEHANDLUNG EINES RELATIVEN UND ABSOLUTEN ANDROGENMANGELS BEIM MANN**

COMBINATION OF DEHYDROEPIANDROSTERONE AND AROMATASE INHIBITORS AND USE OF THIS COMBINATION TO PRODUCE A MEDICAMENT FOR TREATING RELATIVE AND ABSOLUTE ANDROGEN DEFICIENCY IN MEN

ASSOCIATION DE DEHYDRO-EPIANDROSTERONE ET D'INHIBITEURS DE L'AROMATASE ET UTILISATION DE CETTE ASSOCIATION POUR PRODUIRE UN MEDICAMENT EN VUE DE TRAITER UNE CARENCE RELATIVE ET ABSOLUE EN ANDROGENES CHEZ L'HOMME

(84) Benannte Vertragsstaaten:
**AT BE CH DE DK ES FI FR GB GR IE IT LI LU MC NL PT SE**
Benannte Erstreckungsstaaten:
**SI**

(30) Priorität: **06.03.1996 DE 19610645**

(43) Veröffentlichungstag der Anmeldung:
**23.12.1998 Patentblatt 1998/52**

(73) Patentinhaber: **SCHERING AKTIENGESELLSCHAFT 13353 Berlin (DE)**

(72) Erfinder:
- **ELLIESEN, Jörg D-13467 Berlin (DE)**
- **RADLMAIER, Albert D-16727 Bärenklau (DE)**
- **HABENICHT, Ursula D-14167 Berlin (DE)**
- **NEUMANN, Friedmund D-14089 Berlin (DE)**

(56) Entgegenhaltungen:
EP-A- 0 663 402        WO-A-96/09057
GB-A- 2 204 490

- **THE JOURNAL OF STEROID BIOCHEMISTRY, Bd. 32, Nr. 5, 1989, Seiten 625-631, XP002036343 G.H. DECKERS ET AL.: "AROMATASE INHIBITION IN HYPOPHYSECTOMISED FEMALE RATS: A NOVEL ANIMAL MODEL FOR IN VIVO SCREENING"**
- **STEROID BIOCHEMISTRY AND MOLECULAR BIOLOGY, Bd. 38, Nr. 2, 1991, Seiten 181-188, XP002036344 J.A.A. GEELEN ET AL.: "SELECTION OF 19-(ETHYLDITHIO)-ANDROST-4-ENE-3,17-DIONE (ORG 30958): A POTENT AROMATASE INHIBITOR IN VIVO"**
- **ZEITSCHRIFT FÜR ALTERNSFORSCHUNG, Bd. 37, Nr. 3, 1982, Seiten 221-224, XP002036345 M. BOROSS ET AL.: "DIE WIRKUNG DER CHRONISCHEN AMINOGLUTETHIMID-UND DEHYDROEPIANDROSTERON-BEHANDLUNG AUF EINIGE PARAMETER DER OBERSCHENKEL- UND WIRBELKNOCHEN VON RATTEN"**
- **JOURNAL OF CLINICAL ENDOCRINOLOGY AND METABOLISM, Bd. 78, Nr. 6, 1994, Seiten 1360-1367, XP002036346 A.J. MORALES ET AL.: "EFFECTS OF REPLACEMENT DOSE OF DEHYDROEPIANDROSTERONE IN MEN AND WOMEN OF ADVANCING AGE" in der Anmeldung erwähnt**

- **COMP. BIOCHEM. PHYSIOL., Bd. 112B, Nr. 4, 1995, Seiten 613-618, XP002036347 S. MOSLEMI ET AL.: "THE SYNTHESIS OF 19-NORANDROSTENEDIONE FROM DEHYDROEPIANDROSTERONE IN EQUINE PLACENTA IS INHBIBITED BY AROMATASE INHIBITORS 4-HYDROXYANDROSTENEDIONE AND FADROZOLE"**

## Beschreibung

Die Erfindung betrifft die neue Verwendung von Dehydroepiandrosteron (DHEA) in Kombination mit Aromatasehemmern zur Herstellung eines Arzneimittels zur Behandlung eines relativen und/oder absoluten Androgenmangels beim Mann.

Mit zunehmendem Alter kommt es beim Mann zu einer Abnahme der testikulären Androgenproduktion und der Androgenkonzentration im Organismus, sowie, bei Mann und Frau gleichermaßen, zu einer Abnahme der adrenalen Produktion von Dehydroepiandrosteron, einer hormonellen Vorstufe für Androgene und Östrogene. Im Gegensatz zur Situation bei der Frau, in der die Östrogenproduktion innerhalb eines vergleichsweise kurzen Zeitraums auf Kastrationswerte abfällt, handelt es sich beim Mann um ein über Jahrzehnte dauerndes Geschehen und einen nur graduellen Abfall. Es lässt sich dennoch eindeutig nachweisen, dass die Gesamtkonzentration von Testosteron im Serum in der höheren Altersgruppe signifikant gegenüber den Werten bei jungen Männern erniedrigt ist. Durch den gleichzeitig mit dem Alterungsprozess einhergehenden Anstieg des steroidhormonbindenden Globulins (SHBG) sinkt zudem der Anteil des freien, nicht gebundenen und somit biologisch wirksamen Testosteron. Weiterhin fällt auf, dass die Serumspiegel der Östrogene, obwohl sie durch direkte Umwandlung aus Androgenen entstehen, nicht in gleicher Weise altersabhängig absinken. Dadurch wird das hormonelle Milieu deutlich verschoben.

Beim Mann ist das hormonelle Milieu der Sexualsteroide gekennzeichnet durch ein deutliches Übergewicht der Androgene gegenüber den Östrogenen. Während die zirkulierende Hauptkomponente der Androgene, Testosteron, im Serum in Einheiten im Bereich von nmol/l nachgewiesen wird, wird der Östrogen-Gegenspieler, Östradiol, nur im Bereich von pmol/l gemessen. Dieses deutliche Übergewicht der Androgene ist grundsätzlich in der gesamten nachpubertären Lebensspanne nachweisbar, jedoch zeigt sich eine deutlich unterschiedliche Ausprägung dieser Androgendominanz in Abhängigkeit vom Lebensalter. Mit zunehmendem Alter, und deutlicher ausgeprägt bei den über 60jährigen, findet sich eine geringer ausgeprägte Betonung des Androgen-Übergewichts. Publizierte Meßserien, in denen die Relation Serum Testosteron zu Serum Östradiol im Vergleich von jungen zu älteren ($\geq$ 60. Lebensjahr) Männern bestimmt wurde, zeigt Tabelle 1.

Tabelle 1

| Vergleich des T/E$_2$-Verhältnisses im Serum bei jungen und älteren Männern | | | |
|---|---|---|---|
| **Referenz** | **Jung (< 60 J.)** | **Älter (> 60 J.)** | **% $\triangle$ (Abnahme)** |
| Deslypere et al. [1] | 206:1 | 128:1 | - 38 % |
| Pirke & Dörr [2] | 324:1 | 174:1 | - 46 % |
| Baker et al. [3] | 372:1 | 225:1 | - 31 % |
| Murano et al. [4] a.m. p.m. | 155:1 160:1 | 98:1 84:1 | - 37 % - 48 % |

1) Deslypere, J. P. et al., Journal of Clinical Endocrinology and Metabolism, 64, No. 1, 1987

2) Pirke, K. M. & Doerr, P., Acta Endocrinologica, 74 (1973), 792-800

3) Baker, H. W. G. et al., Clinical Endocrinology, 5 (1976), 349-372

4) Murano, E. P. et al., Acta Endocrinologica, 99 (1982), 619-623

Obwohl in den erwähnten Arbeiten das Verhältnis Testosteron zu Östradiol teilweise in deutlich unterschiedlichen Größenordnungen angegeben wird - was auf die verwendeten unterschiedlichen Meßmethoden zurückzuführen ist - zeigt sich bei den älteren Männern eine auffallende Übereinstimmung in den aufgefundenen relativen Abnahmen des Übergewichts von Testosteron um 30 - 50 % gegenüber dem Vorwert bei den jungen Männern.

Das entstehende relative Testosterondefizit kann sich in vielerlei Hinsicht ungünstig auswirken. So wird z. B. vermutet, dass ein durch den Testosteronabfall, bei insgesamt etwa gleichbleibenden Östrogenkonzentrationen, einhergehendes Ungleichgewicht zwischen Androgenen und Östrogenen für die Entstehung der benignen Prostatahyperplasie (BPH) von entscheidender Bedeutung ist. Unabhängig von den Wirkungen der Östrogene, kann allerdings auch das relative Testosterondefizit per se für eine Reihe von altersbedingten Störungen verantwortlich gemacht werden. Zu nennen ist hier die Abnahme der Muskelmasse, einhergehend mit Einschränkung der körperlichen Leistungsfähigkeit, Abnahme der Knochendichte, in Einzelfällen bis zur Osteoporose, Reduktion von Libido und Potenz und psycho-vegetative Störungen. Alle genannten Störungen werden oft unter der Bezeichnung Klimakterium virile zusammengefasst.

Die gängige Behandlung dieses mutmaßlich durch Androgenmangel bedingten Syndroms besteht bisher in der exogenen Zufuhr von Androgenen. Zum Einsatz kommen oral wirksame Androgene und intramuskulär zu applizierende langkettige Testosteronester mit Depoteffekt. Diese Therapieformen sind geeignet, die androgenmangelbedingten Symptome zu bessern, stellen allerdings nur eine unzureichende Annäherung an den physiologischen Zustand her.

Als oral zu verabreichende Substanz wird entweder ein Testosteronderivat, also kein natürliches Testosteron, zugeführt (z. B. Proviron®) oder die Verabreichung geht mit einem von der physiologischen Situation abweichenden überproportionalen Anstieg von Dihydrotestosteron (DHT) einher (z.B. Andriol®). DHT scheint im Gegensatz zu Testosteron der Androgenbestandteil zu sein, der für die Entwicklung der BPH und auch der androgenetischen Alopezie von großer Bedeutung ist.

Bei den Depotformulierungen stellt die ungleichmäßige Freisetzung aus dem Depot ein bisher noch nicht befriedigend gelöstes Problem dar; initial kommt es zu einem deutlich über den Normalbereich hinausreichenden Testosteronanstieg, gegen Ende des Dosierungsintervalls dagegen zu deutlich erniedrigten Testosteronwerten.

Seit langem ist bekannt, dass an dem endokrinen Regelkreis, durch den beim Mann die Androgenspiegel auf konstantem Niveau gehalten werden, neben den Androgenen auch Östrogene beteiligt sind. Durch Gabe pharmakologischer Dosen östrogenwirksamer Substanzen, wie z. B. Diethylstilbestrol, gelingt es bei Patienten mit Prostatakarzinom, die hypophysäre LH-Freisetzung weitgehend zu unterdrücken und die Testosteronspiegel im Serum auf Kastrationsniveau zu senken.

Aus den Erfahrungen mit dem Einsatz von reinen Antiandrogenen bei Patienten mit Prostatakarzinom, die der gleichen Altersgruppe angehören wie Patienten mit Klimakterium virile, lässt sich das Ausmaß des gegenregulatorischen Potentials abschätzen. Wird durch reine Antiandrogene wie Flutamid oder Casodex die zentrale Hemmwirkung von Androgenen unterbunden, so kommt es in dieser Altersgruppe zu einem gegenregulatorischen Anstieg der Serumtestosteronkonzentration um ca. 50 - 60 % gegenüber dem Ausgangswert. Bei einer über Monate andauernden Behandlung zeigten sich allerdings bei den mit reinen Antiandrogenen behandelten Prostatakarzinom-Patienten Hinweise auf ein Nachlassen der Aktivität der Gegenregulation, d.h. die initial deutlich erhöhten Androgenspiegel fallen wieder ab (Lund und Rasmussen, 1988; Mahler und Denis, 1990; Delaere und Van Thillo, 1991).

Auffallend ist, dass die Abnahme der Androgene im Alter nicht über eine Aktivierung des Gegenregulationsmechanismus verhindert wird. Als Ursache hierfür gilt, dass zum einen mit dem Alter die Hodenfunktion allgemein nachlässt, zum anderen aber auch der Feedback-Mechanismus eine gesteigerte Sensitivität für Sexualsteroide aufweist (Deslypere, J. P. et al., Journal of Clinical Endocrinology and Metabolism, 64, No. 1, 1987). Folglich ist davon auszugehen, dass bei älteren Männern im Vergleich mit jüngeren (siehe unten) eine geringer ausgeprägte Gegenregulation vorliegt, weshalb für die langfristige Anwendung mit keiner gegenüber dem Ausgangswert erhöhten Serumandrogenkonzentration zu rechnen ist.

Demgegenüber ist bekannt, dass bei jüngeren Männern durch tägliche Behandlung mit Antiöstrogenen (mit jeweils erheblicher östrogener Partialwirkung), die Testosteronwerte auch in einer Langzeitbehandlung nachhaltig erhöht werden (Treatment of Male Infertility, Springer-Verlag Berlin, Heidelberg, New York 1982; Fuse, H. et al., Archives of Andrology 31 (1993) 139-145).

Antiöstrogene scheinen aufgrund theoretischer Erwägungen zur Behandlung eines relativen Androgenmangels beim Mann wenig geeignet. So hat eine Behandlung mit Antiöstrogenen keinen Einfluss auf den Östrogenspiegel, da diese die Wirkung der Östrogene an deren Rezeptor blockieren. Bei Verwendung von Antiöstrogenen als Rezeptorenblocker führte eine ungenügende Compliance sofort zum ungünstigsten Effekt, da wegen der einsetzenden Gegenregulation die höhere Östrogenkonzentration unmittelbar über die nun freigewordenen Rezeptoren wirken kann.

Ein weiterer Nachteil einer Antiöstrogen-Behandlung ist die Ungewissheit, ob die Blockade der Östrogen-Rezeptoren in allen östrogenabhängigen Geweben und Organen gleich stark ausgeprägt ist und welche Bedeutung die Eigenöstrogenität, wie sie z. B. das bekannteste Antiöstrogen Tamoxifen aufweist, für die Anwendung beim Mann hat.

Comp. Biochem. Physiol. Vol. 112B(4), 1995, Seiten 613 - 618, offenbart ein Kombinationspräparat enthaltend DHEA (Sulfat) und einen Aromatasehemmer in einem salinen Lösemittel zur oralen Verabreichung.

In J. Steroid. Biochem., Vol. 32 (5), 1989, S. 625 - 631 ist die (orale) Behandlung von Ratten mit DHEA-Sulfat, gefolgt von der (subkutanen) Gabe eines Aromatasehemmers (Formestan, Atamestan) beschrieben.

Eine Verwendung im erfindungsgemäßen Sinne ist in den beiden letztgenannten Publikationen, jedoch nicht offenbart.

Die vorliegende Erfindung hat die Aufgabe, geeignete Substanzen bereitzustellen, die einen relativen Androgenmangel (Hypoandrogenismus) beim Mann bei gleichzeitiger Annäherung an das physiologische hormonale Verhältnis von Androgenen zu Östrogenen beheben und die die genannten Nachteile vermeiden.

Diese Aufgabe wird gemäß vorliegender Erfindung durch die Verwendung von Dehydroepiandrosteron (DHEA) und mindestens eines selektiven Aromatasehemmers ausgewählt aus der Gruppe der Verbindungen Atamestan, Formestan, Pentrozol, Arimidex, Fadrozol, CGS 20267, Vorozol zur Herstellung eines Arzneimittels zur Behandlung eines relativen und absoluten Androgenmangels beim Mann, gelöst.

Es wurde festgestellt, dass der Einsatz einer Kombination von DHEA und mindestens eines Aromatasehemmers aus der vorstehenden Gruppe bei der Behandlung eines relativen Androgenmangels bei älteren Männern zu einer langfristigen Erhöhung der Androgenspiegel führt.

Durch graduelle Absenkung der Östrogen-Konzentration wird eine gegenregulatorische Stimulierung der Androgensynthese induziert. Das Ausmaß der Androgenspiegelerhöhung durch alleinige Gabe eines Aromatasehemmers ist jedoch begrenzt durch den gleichzeitig vorherrschenden DHEA-Mangel. Wird der Aromatasehemmer mit einer DHEA-Substitution kombiniert, lässt sich die Steoridbiosynthese der Androgene zuverlässiger und wirksamer auf normale Werte korrigieren. Auch ein absoluter Androgenmangel lässt sich so beheben, ohne dass exogen Androgene zugeführt werden müssen. Die Verwendung der Kombination eines Aromatasehemmers mit einer DHEA-Substitution führt zu einem Synergismus - jeder Wirkstoff für sich allein kann das gewünschte Ziel nicht in dem Maße erreichen, wie die Kombination. Der Limitierung des Aromatase-Wirkprinzips durch den DHEA-Mangel steht die Limitierung einer alleinigen DHEA-Substitution durch die dann beim Mann resultierende, unerwünschte Erhöhung der Östrogenbiosynthese-Rate gegenüber.

Neben der Behandlung eines Hypoandrogenismus beim älteren Mann kann das erfindungsgemäß zu verwendende pharmazeutische Kombinationspräparat in weiteren Indikationen verwendet werden, die sich aus dem Mangel an DHEA und den in der Steroidbiosynthese nachfolgenden Androgenen ergeben.

Die im Alter zunehmende Häufigkeit von Herz/Kreislauferkrankungen, Krebserkrankungen, Stoffwechselerkrankungen, sowie die Infektanfälligkeit und die Verminderung der körperlichen und geistigen Leistungsfähigkeit können direkt oder indirekt auf die nachlassende Hormonproduktion zurückgeführt werden. Die Beseitigung des vorherrschenden Hormonmangels durch das Aromatase + DHEA-Behandlungsprinzip stellt eine wirksame therapeutische Intervention dar zur Prävention und Behandlung dieser typischen Alterserscheinungen.

In diesem Zusammenhang sind allein für das DHEA zahlreiche Wirkungen im Tiermodell und am Menschen beschrieben. Hierzu gehören antiproliferative Effekte, immunmodulatorische Effekte, Wirkungen auf den Kohlenhydrat- und Lipidstoffwechsel, auf das Körpergewicht, die Fettmasse und Muskelmasse, auf die Gerinnung, Wirkungen im Sinne einer Kardioprotektion, Wirkungen auf das zentrale Nervensystem, auf die mitochondriale Zellatmung, sowie eine Schutzwirkung der Zelle vor Sauerstoffradikalbildung.

Durch Aromatasehemmer wird die Bildung von Östrogenen aus deren metabolischen Vorstufen durch Hemmung des Enzyms Aromatase (Hemmung der Biosynthese) verhindert.

Zur Herstellung eines Arzneimittels zur Behandlung eines relativen Androgenmangels (Hypoandrogenismus) beim Mann gemäß vorliegender Erfindung werden die selektiven Aromatasehemmer aus der Gruppe

1-Methyl-androsta-1,4-dien-3,17-dion (DE-A 33 22 285; Atamestan),

4-Hydroxy-4-androsten-3,17-dion (Formestan)

(RS)-5-(4-Cyanphenyl)-5,6,7,8-tetrahydro-imidazo-(1,5$\alpha$)-pyridin, Hydrochlorid (Cancer Res., 48, S. 834-838, 1988; Fadrozol),

4-[Cyan-$\alpha$-(1,2,4-triazol-1-yl)-benzyl]-benzonitril (CGS 20267),

5-[Cyclopentyliden-(1-imidazolyl)-methyl]-thiophen-2-carbonitril (EP-A 0 411 735; Pentrozol),

2,2'-[5-(1H',2',4-Triazol-1-yl-methyl)-1,3-phenylen]-bis(2'-methylpropionitril) (Arimidex) sowie

(6-[1-(4-Chlorphenyl)-1,2,4-triazol-1-yl)-methyl]-1-methyl-1H-benzotriazol, Dihydrochlorid (Vorozol)

verwendet. Unter selektiven Aromatasehemmern sollen solche Verbindungen verstanden werden, die als Substrat für die Aromatase fungieren und in der eingesetzten Dosierung außer der Aromatase keine anderen Enzyme in klinisch relevanter Weise beeinflussen.

Entgegen der Annahme, dass bei Behandlung älterer Männer mit einem Aromatasehemmer über mehrere Monate der gegenregulatorische Impuls nachlassen könnte, zeigen Daten aus länger dauernden Studien, z. B. mit Atamestan an Patienten mit BPH, dass auch nach 24 bis 48 Wochen langer Behandlung weiterhin eine signifikante Erhöhung der Testosteronkonzentration vorliegt.

Die entsprechenden Ergebnisse einer 24wöchigen, vierarmigen Studie im Vergleich zu Placebo (100 mg/d, 300 mg/d und 600 mg/d) zeigt Tabelle 2.

Tabelle 2:

| Testosteronserumkonzentration (ng/ml) unter Atamestan | | | |
|---|---|---|---|
| Tagesdosis | Vorwert | nach 24 Wochen | $\Delta$ % (Median $\pm$ SD) |
| Placebo | 4.13 | 4.18 | 6.00 $\pm$ 27.64 |
| 100 mg | 4.41 | 5.57 | 29.57 $\pm$ 34.70 |
| 300 mg | 4.50 | 6.15 | 40.88 $\pm$ 156.12 |
| 600 mg | 3.78 | 5.40 | 41.19$\pm$ 37.62 |

Tabellen 3 und 4 zeigen die Ergebnisse nach einer 48wöchigen Behandlung.

Tabelle 3:

| Testosteronserumkonzentration (ng/ml) unter Atamestan | | | |
|---|---|---|---|
| **Tagesdosis** | **Vorwert** | **nach 48 Wochen** | **Δ % (X ± SD)** |
| Placebo | 4,6 | 4,1 | -0,1 ± 43,1 |
| 400 mg | 4,2 | 5,4 | 42,9 ± 53,5 |

Tabelle 4:

| Testosteronserumkonzentration (ng/ml) unter Atamestan | | | |
|---|---|---|---|
| **Tagesdosis** | **Vorwert** | **nach 48 Wochen** | **Δ % (X ± SD)** |
| Placebo | 4,6 | 4,6 | 2,8 ± 26,9 |
| 100 mg | 5,1 | 5,9 | 19,0 ± 36,8 |
| 300 mg | 4,7 | 6,6 | 41,7 ± 46,4 |

Durch graduelle Absenkung der Östrogenkonzentration wird eine gegenregulatorische Stimulierung der Androgensynthese induziert. Gewissermaßen kommt es zu einer endogenen Testosteronsubstitution, wodurch die Androgen-/Östrogen-Balance wieder in den "jugendlichen" Bereich zurückgeführt wird. Dies belegen die Ergebnisse der längerfristigen Behandlung älterer Männer (Durchschnittsalter über 60 Jahre) mit dem selektiven Aromatasehemmer Atamestan.

In mehreren klinischen Studien wurde Männern dieser Altersgruppe zur Behandlung einer bestehenden BPH Atamestan in unterschiedlichen Dosierungen und über Zeiträume bis zu 48 Wochen verabreicht. Die Ergebnisse zeigen, dass unter Atamestan-Behandlung für Patientenkollektive eine signifikante Verschiebung des Testosteron-/Östradiol-Verhältnisses zugunsten von Testosteron eintritt. Tabelle 5 gibt die Testosteron-/Östrogen-Ratio vor und nach Atamestan-Gabe für Patientenkollektive aus 4 Studien und 7 Behandlungsgruppen wieder.

Tabelle 5

| Änderungen des T/$E_2$-Verhältnisses unter Atamestan | | | |
|---|---|---|---|
| **Behandlungsgruppe** | **Vorwert** | **Behandlung (Zeitpunkt)** | **% Δ** |
| 100 mg | 248:1 | 418:1 (48. Woche) | + 41 % |
| 300 mg | 236:1 | 440:1 (48. Woche) | + 46 % |
| 400 mg | 207:1 | 454:1 (48. Woche) | + 54 % |
| 200 mg t.i.d. | 116:1 | 214:1 (8. Woche) | + 46 % |
| 100 mg | 196:1 | 376:1 (24. Woche) | + 48 % |
| 300 mg | 199:1 | 473:1 (24. Woche) | + 58 % |
| 600 mg | 170:1 | 439:1 (24. Woche) | + 61 % |

Die Atamestangabe führt durchweg zu einer Neueinstellung der Testosteron/Östradiol-Balance zugunsten der androgenen Komponente. Diese Wirkung war über den gesamten Beobachtungszeitraum bis zu maximal 48 Wochen Behandlung nachweisbar. Obwohl die periphere Östrogen-Absenkung in den täglichen Dosierungen 100 mg - 600 mg gleich stark ausgeprägt war, zeigt sich ein Trend zur stärkeren Betonung des androgenen Anteils bei hohen Dosierungen.

Unter der Annahme, dass die in der Altersgruppe der Patienten über 60 Jahren gegenüber den jüngeren Jahrgängen um 30 - 50 % erniedrigte Testosteron-Dominanz (relativer Androgenmangel) das Beschwerdebild des "männlichen Klimakteriums" verursacht, ist somit das Ziel, das ursprüngliche "Kräfteverhältnis" zwischen Androgenen und Östrogenen wiederherzustellen, durch die Gabe eines, vorzugsweise selektiven, Aromatasehemmers durch Stimulation der endogenen Testosteron-Substitution ohne Notwendigkeit einer exogenen Zufuhr von Androgenen erreicht. Anhand der Maßgabe, dass die im Alter vorherrschende Relation das Ergebnis einer 30 - 50 %igen Absenkung gegenüber den jugendlichen Werten ist, d.h. immer noch 50 - 70 % des Vorwertes beträgt, lässt sich für jeden einzelnen

EP 0 885 005 B1

Patienten der entsprechende "jugendliche" Vorwert kalkulieren. Ein 70jähriger Patient mit einem Testosteron-/Östradiol-Verhältnis von 230:1 muss demnach auf eine neue Balance im Bereich zwischen 229:1 bis 460:1 eingestellt werden, damit eine vorangegangene 30- bzw. 50 %ige Abnahme ausgeglichen ist. Tabelle 6 zeigt das Ergebnis einer derartigen Kalkulation für die Patientenkollektive der in Tabelle 4 aufgeführten Atamestan-Studien.

Tabelle 6

| Vergleich zwischen kalkuliertem "jugendlichen" T/E$_2$-Bereich und ge- messenen Werten unter verschiedenen Atamestan-Tagesdosierungen | | | |
|---|---|---|---|
| Tagesdosis | Vorwert | Kalkulierter Zielbereich zum Ausgleich einer 30-50 %igen Absenkung | Erreichter Wert unter Atamestan |
| 100 mg | 248:1 | 354:1 → 496:1 | 418:1 |
| 300 mg | 236:1 | 337:1 → 472:1 | 440:1 |
| 400 mg | 207:1 | 296:1 → 414:1 | 454:1 |
| 100 mg | 196:1 | 280:1 → 392:1 | 376:1 |
| 300 mg | 199:1 | 284:1 → 398:1 | 473:1 |
| 600 mg | 170:1 | 243:1 → 340:1 | 439:1 |

Mit einer Tagesdosis von 100 mg ist der Zielbereich im allgemeinen gut getroffen. Bei höheren Dosierungen hingegen liegt das Ergebnis etwas oberhalb des Zielbereichs.

Durch Messung der Serumkonzentration von Testosteron und Östradiol kann somit frühzeitig kontrolliert werden, ob die angestrebte Hormonbalance erreicht wurde und gegebenenfalls eine Dosisanpassung vorgenommen werden muss.

Gegenüber dieser vorstehend beschriebenen Behandlung eines Hypoandrogenismus mit mindestens einem Aromatasehemmer alleine (nicht veröffentlichte DE P 44 35 368.5), führt eine Behandlung mit DHEA und mindestens einem selektiven Aromatasehemmer, aus der Gruppe der Verbindungen Atamestan, Formestan, Pentrozol, Arimidex, Fadrozol, CGS 20267, Vorozol zu einem synergistischen Effekt.

Der wichtigste Vorläufer der Androgene Testosteron und Dihydrotestosteron (DHT) ist Dehydroepiandrosteron (DHEA), das das menschliche Sexualsteroid mit der höchsten täglichen Syntheserate darstellt. Es ist bekannt, dass die DHEA-Plasmaspiegel im Alter bis auf 20% der Maximalwerte in der Adoleszenz absinken.

Die Kombination von DHEA mit mindestens einem Aromatasehemmer weist beispielsweise die folgenden Vorteile auf:

- Durch den synergistischen Effekt wird eine Dosisreduktion des Aromatasehemmers möglich unter Beibehaltung des moderaten Anstiegs von Testosteron und DHT. Dies führt zur Vermeidung von Nebenwirkungen, welche durch beide aktiven Substanzen verursacht werden können.

- Durch den synergistischen Effekt kommt es bei gleichbleibender Dosis des Aromatasehemmers zu einer deutlicheren und zuverlässigeren Erhöhung von Testosteron und DHT.

- Bei absolutem Androgenmangel wird dieser durch die Kombination des Aromatasehemmers mit DHEA im Sinne eines Synergismus wirksam behandelt. Dabei wird weiterhin eine Zufuhr exogener Androgene mit den damit verbundenen Nachteilen vermieden.

- Da auch DHEA und die in der Androgenbiosynthese nachfolgenden Steroidhormone eigene pharmakologische Wirkung besitzen, können diese bei gleichzeitiger Kombination mit einem Aromatasehemmer genutzt werden, ohne dass es gleichzeitig zur Zunahme der Östrogenbiosynthese kommt, die beim Mann unerwünscht ist.

Neben der klassischen physiologischen Rolle als Hormonvorstufe in der Sexualsteroid-Biosynthese sind zahlreiche Wirkungen des DHEA im Tiermodell und am Menschen beschrieben. Hierzu gehören antiproliferative Wirkungen, immunmodulatorische Wirkungen, eine gewichtsreduzierende Wirkung mit Reduktion der Fettmasse zugunsten der Muskelmasse, Wirkung auf Kohlenhydrat- und Lipidstoffwechsel, Wirkungen auf die Gerinnung, eine kardioprotektive Wirkung, Wirkungen auf das zentrale Nervensystem, auf die mitochondriale Zellatmung, sowie eine Schutzwirkung für die Zelle vor Sauerstoffradikalbildung. In einer Publikation von Yen et al. (Effects of replacement dose of dehydroepiandrosterone in men and women of advancing age. J. Clin Endocrinol Metab 78: 1360-1367,1994) werden Effekte auf Wachstumsfaktoren beschrieben, die in Zusammenhang gebracht werden können mit der physiologischen Zellalterung. Zusätzlich wird dort ein positiver Effekt von DHEA auf das physische und psychische Wohlbefinden beschrieben.

DHEA wird in der Kombination in einer täglichen Menge von 10 mg bis 100 mg, vorzugsweise von 20 mg bis 60 mg, eingesetzt, wenn der Substitutionsgedanke im Vordergrund steht. Bei therapeutischem Einsatz von DHEA unter gleichzeitiger Gabe eines selektiven Aromatasehemmers zur Vermeidung einer Erhöhung der Östrogenspiegel, liegen DHEA-Dosierungen in der Größenordnung von bis zu 1000 mg im Rahmen vorliegender Erfindung.

Im allgemeinen werden täglich 25 bis 500 mg, vorzugsweise 50 bis 250 mg, Atamestan oder eine biologisch äquieffektive Menge eines anderen selektiven Aromatasehemmers zur Behandlung eines relativen oder absoluten Androgenmangels beim Mann in der erfindungsgemäßen Kombination eingesetzt.

DHEA und die selektiven Aromatasehemmer können z. B. oral oder parenteral appliziert werden. Für die bevorzugte orale Applikation kommen insbesondere Tabletten, Dragèes, Kapseln, Pillen, Suspensionen oder Lösungen infrage, die in üblicher und dem Fachmann geläufiger Weise mit den in der Galenik für die Formulierung von oral zu applizierenden Aromatasehemmern üblichen Zusätzen und Trägersubstanzen hergestellt werden können.

DHEA und der selektive Aromatasehemmer können gemeinsam oder getrennt formuliert werden. Bei getrennter Formulierung ist auch eine sequentielle Gabe vorstellbar.

Das erfindungsgemäße Arzneimittel enthält als Wirkstoff je Dosiseinheit den Aromatasehemmer Atamestan in einer Dosierung von 25 bis 250 mg neben den üblichen Hilfs-, Träger- und/oder Verdünnungsmitteln oder andere selektive Aromatasehemmer in biologisch äquieffektiver Dosierung.

DHEA ist in der gemeinsamen oder in einer getrennten Dosiseinheit in einer Menge von 5 bis 200 mg enthalten neben den üblichen Hilfsstoffen.

Ein typische Zusammensetzung für eine Formulierung des Aromatasehemmers Atamestan mit DHEA als Tablette ist im nachstehenden Beispiel 2 wiedergegeben.

**Beispiele**

Beispiel 1.

Im Rahmen einer klinischen Studie werden täglich 50 mg DHEA und 100 mg Atamestan oral verabreicht. Es handelt sich um 100 ältere Herren im Alter von 70 oder darüber, die nach einem Zufallsverfahren in vier Behandlungsgruppen eingeteilt wurden. Sie erhalten entweder DHEA (50mg/Tag) oder Atamestan (100 mg/Tag) oder DHEA + Atamestan oder aber Placebo für 36 Wochen und werden anschließend noch ein halbes Jahr nachbeobachtet.

Beispiel 2.

| 100,0 mg | 1-Methyl-androsta-1,4-dien-3,17-dion |
|---|---|
| 50,0 mg | DHEA |
| 140,0 mg | Laktose |
| 70,0 mg | Maisstärke |
| 2,5 mg | Poly-N-Vinylpyrrolidon 25 |
| 2,0 mg | Aerosil |
| 0,5 mg | Magnesiumstearat |
| 365,0 mg | Gesamtgewicht der Tablette, die in üblicher Weise auf einer Tablettenpresse hergestellt wird. |

Bei Verwendung von DHEA und selektiven Aromatasehemmern zur Behandlung des Klimakterium virile wird die Östrogenkonzentration wirkungsvoll gesenkt und eine unerwünschte Erhöhung der Östrogenspiegel durch die DHEA-Substitution wirkungsvoll unterdrückt. Die leichte Steuerbarkeit der Therapie zeichnet die Behandlung mit DHEA und einem selektiven Aromatasehemmer zur Stimulation der endogenen Testosteronproduktion gegenüber dem Einsatz mit Antiöstrogenen aus. Wie bereits ausgeführt, ist mit Antiöstrogenen eine prospektive Steuerung der Therapie durch frühzeitige Messung

**Patentansprüche**

**1.** Verwendung von Dehydroepiandrosteron (DHEA) und mindestens eines selektiven Aromatasehemmers ausgewählt aus der Gruppe der Verbindungen Atamestan, Formestan, Pentrozol, Arimidex, Fadrozol, CGS 20267, Vorozol zur Herstellung eines Arzneimittels zur Behandlung eines relativen und absoluten Androgenmangels beim Mann.

**Claims**

1. Use of dehydroepiandrosterone (DHEA) and at least one selective aromatase inhibitor selected from the group consisting of the compounds atamestan, formestan, pentrozole, arimidex, fadrozole, CGS 20267, vorozole for the production of a medicament for the treatment of a relative and absolute androgen deficiency in man.

**Revendications**

1. Utilisation de la déshydroépiandrostérone (DHEA) et d'au moins un inhibiteur d'aromatase sélectif choisi parmi le groupe constitué des composés Atamestane, Formestane, Pentrozole, Arimidex, Fadrozole, CGS 20267 et Vorozole, pour la préparation d'un médicament destiné au traitement d'une déficience androgénique relative et absolue chez l'homme.